## Europäisches Patentamt
## European Patent Office
## Office européen des brevets

(19)

(11) Numéro de publication: **0 032 467**
**A1**

# DEMANDE DE BREVET EUROPEEN

(12)

(21) Numéro de dépôt: **81400017.0**

(22) Date de dépôt: **08.01.81**

(51) Int. Cl.³: **C 07 C 143/12**
**C 07 C 143/18**

(30) Priorité: **10.01.80 FR 8000521**

(43) Date de publication de la demande:
**22.07.81 Bulletin 81/29**

(84) Etats contractants désignés:
**BE DE GB IT NL**

(71) Demandeur: **DUMAS et INCHAUSPE Societé en nom collectif dite Building des Pyrénées 1-A**
**Avenue de Lattre de Tassigny**
**F-64000 Pau(FR)**

(72) Inventeur: **Inchauspé, Nicolas Désiré**

**F-64370 Arthez de Bearn(FR)**

(74) Mandataire: **Lepeudry-Gautherat, Thérèse et al,**
**Cabinet Armengaud Jeune Casanova, Akerman,**
**Lepeudry**
**23 boulevard de Strasbourg F-75010 Paris(FR)**

(54) Procédé de préparation en continu de sulfonates dérivés de molécules à longue chaîne alkyle et plus particulièrement d'esters gras.

(57) Procédé de préparation en continu de solfonates dérivés d'esters gras à longue chaîne.

Selon l'invention on soumet le produit de départ (5), à l'action d'un agent sulfonant (10), on dégaze (2), le produit de sulfonation ainsi obtenu (6) et on le traite (3) avec du méthanol légèrement aqueux pour obtenir d'une part une phase ester contenant le produit de départ non sulfoné que l'on recycle (12) dans l'étape de sulfonation, et d'autre part une phase méthanol qui contient les acides sulfoniques recherchés et qui est soumise à une évaporation (4) permettant de séparer d'une part le méthanol et d'autre part lesdits acides sulfoniques.

EP 0 032 467 A1

./...

La présente invention a pour objet un procédé de préparation en continu de sulfonates dérivés de molécules à longue chaîne alkyle c'est-à-dire contenant au moins 11 groupes $CH_2$ et les produits ainsi obtenus. L'invention concerne plus particulièrement la sulfonation des esters gras. Par esters gras on entend notamment les esters saturés alkyliques d'origine naturelle, c'est-à-dire obtenus à partir d'huiles végétales telles que l'huile de palme, de soja ou de colza, ou à partir de graisses animales telles que le suif. Bien entendu le procédé de l'invention s'applique également aux esters gras d'origine synthétique.

Les produits obtenus selon l'invention à partir des esters gras sont particulièrement intéressants comme agents de surface car ils sont biodégradables tout en étant aussi détergents que les sulfonates obtenus à partir de fractions pétrolières ; ils ont de plus la propriété de séquestrer le calcium et les autres ions divalents de l'eau, ce qui permet d'éviter l'utilisation de phosphates, laquelle est interdite dans de nombreux pays en raison des risques d'eutrophisation.

Pour sulfoner les esters gras on a essayé d'utiliser le procédé de sulfonation par l'anhydride sulfurique $SO_3$ gazeux préparé préalablement par oxydation catalytique d'anhydride sulfureux. Toutefois ce procédé, qui est utilisé industriellement pour sulfoner les alkyl-aryles ou les $\alpha$-oléfines, ne donne pas de résultats satisfaisants pour la sulfonation des esters gras. En effet la molécule de ces esters ne contient qu'un carbone faiblement actif, le carbone en position $\alpha$ du carbone acide, de sorte que la fixation de $SO_3$ qui est spontanée, exothermique et totale dans le cas des alkylaryles et des $\alpha$-oléfines, est lente et incomplète dans le cas des esters gras sulfonés. Une description des difficultés rencontrées pour sulfoner des esters gras par l'anhydride sulfurique est donnée dans l'article de Stein et Baumann, JOACS, Vol. 52, pages 323-329 (1975) et dans celui de Kapur, Solomon et Bluestein, JOACS, Vol. 55, page 549 (1978).

On a par ailleurs proposé dans le brevet allemand DT 907053 d'opérer une sulfonation d'acides gras et de leurs dérivés à l'aide d'un mélange d'anhydride sulfureux et d'oxygène agissant sous irradiation ultraviolette. Selon ce brevet on soumet ainsi à une sulfo-oxydation notamment du laurate de méthyle pour obtenir un produit de sulfonation que l'on hydrolyse, avant de filtrer l'acide laurique insulfoné, et que l'on peut ensuite extraire au butanol avant neutralisation à la soude. D'autre part les chercheurs russes Nametkin et al. ont publié une étude de laboratoire faite sur la sulfo-oxydation d'esters gras synthétiques (Neftekhimiya, vol 15, N° 5, 1975, pages 760-762). Ces auteurs, qui évidemment ne se préoccupent pas de définir un procédé industriel, extraient les acides obtenus par du carbonate de baryum, le sulfonate de baryum étant ensuite passé sur une colonne échangeuse d'ions pour donner le sulfonate de sodium.

Enfin dans le domaine de l'extraction des acides sulfoniques fabriqués à partir d'huiles minérales on a utilisé du méthanol soit pur soit associé à l'eau. Ainsi dans le brevet des Etats-Unis d'Amérique N° 3.225.086 on ajoute le méthanol après l'eau de façon que les proportions finales soient de 33 % d'eau et 66 % de méthanol. Ce procédé ne peut s'appliquer à des esters gras qui seraient bien entendu hydrolysés. Dans le brevet britannique N° 672.736 on procède à une première extraction par un mélange méthanol/eau où la proportion d'eau est supérieure à 20 % (d'où risque d'hydrolyse) puis on élimine ensuite l'acide sulfurique par une essence légère. Finalement dans le brevet des Etats-Unis d'Amérique N° 3.083.146 le traitement au méthanol n'est pas une extraction des produits de sulfonation mais est appliqué à une phase acide pure dans le but de diminuer la teneur en acide sulfurique. En fait ces différents procédés ne se préoccupent pas d'extraire tous les acides formés dans l'huile ; ils ne réalisent donc qu'un seul traitement, ce qui donne dans la phase extraite une composition d'acide différente de la composition obtenue dans l'huile et n'est donc pas favorable du point de vue économique.

La Demanderesse a maintenant découvert selon l'invention qu'il était possible d'effectuer une sulfonation en continu de molécules à longue chaîne alkyle, et en particulier d'esters gras, en extrayant le produit sulfoné à l'aide de méthanol faiblement aqueux par traitement répété et en recyclant le produit insulfoné. Ce procédé s'avère particulièrement intéressant dans le cas des esters gras.

En effet lorsque l'on soumet des esters gras à une sulfonation par l'anhydride sulfurique ou par un mélange d'anhydride sulfureux et d'oxygène sous irradiation on constate que la fraction d'ester sulfoné est faible, par rapport à la quantité d'ester n'ayant pas réagi. Pour rendre un procédé de sulfonation d'ester gras industriellement rentable il s'avère donc nécessaire soit de prévoir plusieurs réacteurs de sulfonation en cascade - mais cela suppose un appareillage coûteux -, soit de recycler dans l'étape de sulfonation l'ester n'ayant pas réagi, et pour cela il faut trouver un agent d'extraction approprié pour l'ester sulfoné.

Or, on ne peut employer de l'eau comme extracteur car l'eau hydrolyse l'ester, d'autant mieux que l'hydrolyse est facilitée par l'acidité du milieu. Cette hydrolyse est du reste facilement observée sur le produit neutralisé à la soude qui présente une bande d'absorption IR à 1560 cm$^{-1}$ correspondant au carboxylate de sodium. D'autre part on ne peut pas employer du méthanol pur car aux températures supérieures à 30°C auxquelles on est obligé de travailler, le méthanol dissout complètement les corps gras. Par contre, la Demanderesse a découvert de façon surprenante que du méthanol légèrement aqueux constituait un excellent agent d'extraction.

Pour établir quantitativement les propriétés extractrices pour l'ester du mélange méthanol-eau, on a étudié la miscibilité des mélanges esters gras - méthanol - eau. Pour cela, un mélange initial de composition donnée est agité et l'on sépare les deux phases obtenues après séparation par décantation. On analyse la composition de chacune des phases, par une distillation sous vide qui entraîne l'eau et le méthanol, suivie d'une chromatographie qui sépare l'eau et le méthanol.

Les résultats sont donnés dans les tableaux suivants :

## TABLEAU I

Mélange eau, méthanol et huile de palme hydro-génée et méthylée   % poids des constituants, à 35°C

| dans la phase méthanol obtenue après décantation | | | dans la phase ester obtenue après décantation | | |
|---|---|---|---|---|---|
| Ester | Méthanol | Eau | Ester | Méthanol | Eau |
| 25,8 | 73,1 | 1,2 | 69,5 | 29,9 | 0,6 |
| 11,5 | 83,2 | 5,4 | 83,4 | 16,6 | 0,1 |
| 6,2 | 84,8 | 9 | 89,4 | 10,5 | 0,2 |
| 3,8 | 79,1 | 17,2 | 96,2 | 3,7 | 0,1 |

## TABLEAU II

Mélange eau, méthanol et suif hydrogéné et méthylé   % poids des constituants, à 35°C

| Phase méthanol | | | Phase ester | | |
|---|---|---|---|---|---|
| Ester | Méthanol | Eau | Ester | Méthanol | Eau |
| 21,6 | 77,2 | 1,2 | 73,6 | 26,0 | 0,4 |
| 13,9 | 83,0 | 3,1 | 80,1 | 19,8 | 0,1 |
| 8,1 | 82,8 | 9,1 | 88,3 | 11,5 | 0,2 |
| 4,5 | 76,9 | 18,6 | 92,8 | 7,2 | 0,1 |

Ces tableaux font bien apparaître que sous l'action d'une faible quantité d'eau (comprise entre 1 et 3 % en poids total), le mélange initialement homogène de méthanol et d'ester se sépare en deux phases, dont l'une contient surtout du méthanol et l'autre surtout de l'ester. On voit aussi que l'eau se retrouve presque entièrement dans la phase méthanol ; tout se passe par conséquent comme si le mélange ternaire ester-méthanol-eau se comportait comme un mélange binaire de deux liquides peu miscibles : ester et méthanol aqueux.

On a ensuite procédé à des tests d'extraction d'acides sur des mélanges obtenus par photosulfonation qui contenaient environ 20 % d'acides sulfoniques et 80 % d'ester insulfoné. Il faut noter que la photosulfonation ne donne pas seulement des acides monosulfoniques (mA) mais aussi des acides disulfoniques (dA) et de l'acide sulfurique ($SO_4$) autour de la proportion moyenne 78/15/7 pour cent en poids. Pour déterminer la proportion de chacun de ces acides, on utilise deux méthodes analytiques bien connues : la titrimétrie au bleu de méthylène EPTON, et la potentiométrie en milieu anhydre.

Le tableau III ci-après donne les résultats significatifs obtenus pour l'extraction d'acides sulfoniques par le méthanol aqueux qui caractérise le procédé selon l'invention. On a réalisé sur le mélange initial, désigné par F, trois extractions successives selon la méthode bien connue de l'extraction liquide/liquide à cocourant, c'est-à-dire en ajoutant trois fois du méthanol aqueux vierge, une première fois à F, une seconde fois à la phase surnageante $R_1$, une troisième fois à la phase surnageante obtenue $R_2$, de façon à obtenir une phase finale surnageante $R_3$ qui ne contient pratiquement plus d'acides sulfoniques. On obtient à la première décantation un extrait $E_1$, à la seconde un extrait $E_2$, à la troisième un extrait $E_3$. Le mélange des trois extraits donne un extrait total $E_T$, qui, après évaporation du méthanol, donne l'extrait total sec $E_{Tsec}$. Les mélanges avant décantation en deux phases sont représentés par $M_1$, $M_2$ et $M_3$. La densité est représentée par d. La totalité des acides est représentée par A et les esters gras non sulfonés par FE.

Dans ce tableau ainsi que dans ce qui suit, les pourcentages indiqués sont des pourcentages pondéraux.

(voir tableau III page suivante)

## TABLEAU III

### Extraction à 50°C des acides sulfoniques par du méthanol légèrement aqueux

| $\Omega$ | m(g) | %mA | %dA | %SO$_4$ | %A | %MW | %FE | mA(g) | dA(g) | SO$_4$(g) | MW(g) | FE(g) | d |
|---|---|---|---|---|---|---|---|---|---|---|---|---|---|
| F | 51,7 | 14 | 4 | 0,6 | 18,6 | O | 81,4 | 7,3 | 2,1 | 0,3 | O | 42,1 | 0,894 |
| M$_1$ | 55,6 | 13 | 3,8 | 0,6 | 17,4 | 7 | 75,6 | 7,3 | 2,1 | 0,3 | 3,9 | 42,1 | 0,890 |
| R$_1$ | 47,7 | 8,7 | 0,1 | 0,2 | 9 | 3,7 | 87,3 | 4,1 | O | 0,1 | 1,8 | 41,7 | 0,868 |
| E$_1$ | 7,9 | 39,4 | 26,1 | 2,8 | 68,3 | 26,9 | 4,8 | 3,1 | 2,1 | 0,2 | 2,1 | 0,4 | 1,014 |
| M$_2$ | 51,3 | 8,1 | 0,1 | 0,2 | 8,4 | 10,5 | 81,1 | 4,1 | O | 0,1 | 5,4 | 41,7 | 0,865 |
| R$_2$ | 46,5 | 4,9 | O | 0,1 | 5 | 7,2 | 87,8 | 2,3 | O | 0,1 | 3,4 | 40,9 | 0,862 |
| E$_2$ | 4,8 | 39,1 | 0,4 | 1,1 | 40,6 | 42,9 | 16,5 | 1,9 | O | 0,1 | 2 | 0,8 | 0,889 |
| M$_3$ | 50,2 | 4,5 | O | 0,1 | 4,6 | 13,9 | 81,5 | 2,3 | O | 0,1 | 7 | 40,9 | 0,859 |
| R$_3$ | 44,9 | 1,9 | O | O | 1,9 | 8,1 | 90 | 0,9 | O | O | 3,6 | 40,4 | 0,859 |
| E$_3$ | 5,3 | 26 | O | 0,6 | 26,6 | 63,3 | 10,1 | 1,4 | O | O | 3,3 | 0,5 | 0,864 |
| E$_T$ | 18 | 35,4 | 11,6 | 1,7 | 48,7 | 41,8 | 9,5 | 6,4 | 2,1 | 0,3 | 7,5 | 1,7 | 0,937 |
| E$_{Tsec}$ | 10,5 | 60,9 | 20 | 2,9 | 83,8 | O | 16,2 | 6,4 | 2,1 | 0,3 | O | 1,7 | 1,021 |

7

0032467

Ce tableau montre que le produit final obtenu $E_T$ en mélangeant les trois extraits $E_1$, $E_2$ et $E_3$ a une composition en acides très voisine de la composition initiale du mélange photosulfoné, et que, après évaporation du méthanol, ce produit contient environ 84 % d'acide total et 16 % d'insulfoné, tandis que le mélange photosulfoné initial contenait 18 % d'acide total et 82 % environ d'insulfoné.

On s'aperçoit que plusieurs étapes d'extraction sont nécessaires, car l'acide disulfonique est plus soluble que les autres et passe tout entier dans la phase méthanolique à la première extraction, ce qui donne un premier extrait enrichi en acide disulfonique, ce qui n'est généralement pas souhaitable.

Des résultats analogues voire meilleurs peuvent être obtenus par la technique connue d'extraction à contre-courant.

Le produit final obtenu est de couleur rouille, et non pas noir comme c'est le cas dans la plupart des autres procédés de sulfonation : il ne nécessite donc pas un traitement spécial pour être blanchi. L'ester résiduel qu'il contient n'est pas gênant, car le produit est ensuite neutralisé en sulfonate et, dans cette même opération, l'ester est transformé en savon, ce qui contribue au pouvoir détergent de l'ensemble. Les disulfonates ont des propriétés solubilisantes analogues à celles des toluènesulfonates et le sulfate de sodium représente une charge neutre dans la formulation du détergent.

L'invention concerne donc un procédé continu de sulfonation de molécules à longue chaîne, caractérisé en ce qu'après avoir soumis le produit de départ à l'action d'un agent sulfonant, on extrait le produit de sulfonation ainsi obtenu avec du méthanol légèrement aqueux pour obtenir d'une part une phase contenant le produit de départ non sulfoné que l'on recycle dans l'étape de sulfonation, et d'autre part, une phase méthanol qui est soumise à une évaporation permettant de séparer d'une part le méthanol et d'autre part les dérivés sulfoniques.

Plus particulièrement l'invention concerne un procédé continu de sulfonation d'esters gras caractérisé en ce qu'après avoir soumis un ester gras, ou un mélange d'esters gras, à l'action d'un agent sulfonant, on extrait le produit de sulfonation ainsi obtenu par traitement répété avec du méthanol légèrement aqueux pour obtenir d'une part une phase ester contenant l'ester ou les esters de départ non sulfonés que l'on recycle dans l'étape de sulfonation et, d'autre part, une phase méthanol qui contient les acides gras et qui est soumise à une évaporation permettant de séparer d'une part le méthanol et d'autre part les acides sulfoniques.

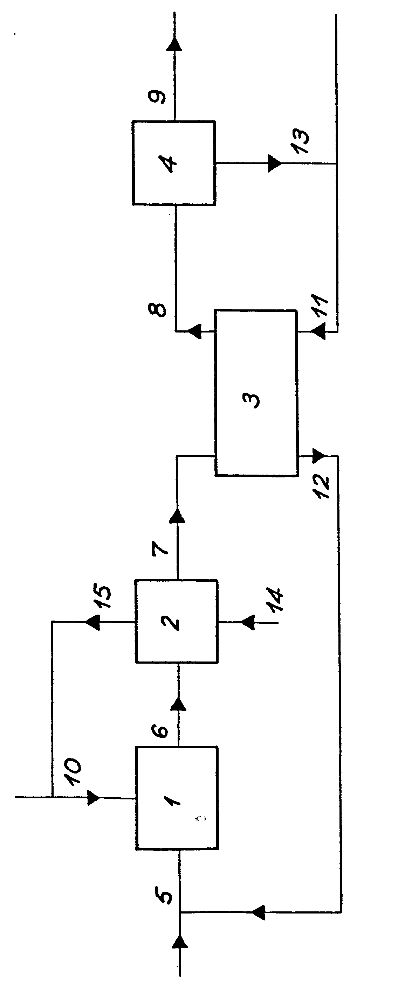

L'invention sera mieux comprise à la lecture de la description qui va suivre d'un exemple de réalisation et en se référant au dessin annexé sur lequel la figure unique représente schématiquement un appareillage destiné à la mise en oeuvre du procédé de l'invention.

Conformément à cette figure, l'ester gras ou le mélange d'esters gras est introduit dans le réacteur de sulfonation 1 par la canalisation 5. L'agent sulfonant est introduit par la canalisation 10 dans le réacteur 1 où il est dispersé dans le milieu réactionnel par tout moyen d'injection gaz/liquide approprié. On soutire en continu du réacteur le produit de sulfonation obtenu qui contient essentiellement la fraction sulfonée de l'ester initial et son complément insulfoné que l'on fait passer par la canalisation 6 dans un dégazeur 2 où un courant d'air d'azote ou d'oxygène introduit par 14 élimine l'agent sulfonant dissout, qui est recyclé dans le réacteur 1 par la canalisation 15. Le produit de sulfonation dégazé est envoyé par la canalisation 7 à un extracteur 3 où le solvant extracteur, c'est-à-dire le méthanol légèrement aqueux est introduit par la canalisation 11. L'ester gras insulfoné (raffinat) issu de l'extracteur est renvoyé par la canalisation 12 au réacteur 1, tandis que l'extrait contenant les acides gras et le méthanol est envoyé par la canalisation 8 à un vaporiseur 4. Le méthanol dégazé par le vaporiseur est recyclé par 13 sur l'extracteur et l'on obtient en 9 à la sortie du vaporiseur, les acides sulfoniques recherchés.

Pour réaliser la sulfonation on opère de préférence par sulfo-oxydation sous irradiation. Pour cela on introduit dans le réacteur 1 un mélange d'anhydride sulfureux et d'oxygène dans un rapport $SO_2/O_2$ égal ou supérieur à 2, le réacteur contenant une lampe, par exemple une lampe au mercure émettant un rayonnement UV compris entre 350 et 450 nm. L'intérêt d'opérer par sulfo-oxydation sous irradiation est que l'on peut mettre en oeuvre le procédé à des températures inférieures à 40°C, ce qui évite de détériorer le produit. Habituellement on opère à une température de l'ordre de 35°C, à laquelle les esters gras sont liquides.

On peut également utiliser comme agent sulfonant l'anhydride sulfurique.

De préférence, on utilise, selon l'invention, pour réaliser l'extraction, une quantité de méthanol aqueux comprise entre la moitié et le double de la quantité de produit de sulfonation sur lequel cette extraction est réalisée. Le méthanol utilisé contient de préférence au plus 20 % et plus particulièrement entre 1 et 5 % en poids d'eau.

Cette extraction liquide/liquide est réalisée à plusieurs étages et de préférence à au moins trois étages. On obtient de bons résultats en effectuant par exemple une extraction à cocourant à trois étages ou à contre-courant à cinq étages. L'avantage de la technique à contre-courant est que bien que l'on soit obligé de prévoir un nombre d'étages plus élevé, les quantités de méthanol aqueux utilisé sont plus faibles.

Comme indiqué plus haut le procédé de l'invention s'applique aux esters gras d'origine naturelle ou synthétique et plus particulièrement aux huiles et graisses animales et végétales ayant subi un traitement d'hydrogénation et de méthylation. On peut ainsi sulfoner conformément au procédé de l'invention du stéarate de méthyle commercial, du suif hydrogéné et méthylé ou de l'huile de palme hydrogénée et méthylée. Le stéarate de méthyle commercial, fondant à 30°C, est du stéarate de méthyle pratiquement pur et correspond assez bien à l'huile de soja ou de colza (non érucique) hydrogénée et méthylée. Le suif hydrogéné et méthylé commercial, fondant à 27°C, comprend environ 66 % de stéarate de méthyle, 27 % de palmitate de méthyle, le reste étant constitué par du myristate de méthyle ou des esters à chaîne plus courte. L'huile de palme hydrogénée et méthylée commerciale comprend environ 60 % de stéarate de méthyle et 40 % de palmitate de méthyle, et peut contenir également jusqu'à 7 ou 8 % de laurate de méthyle, pour les mêmes proportions relatives de stéarate/palmitate.

Conformément à l'invention on obtient des résultats particulièrement intéressants en utilisant un produit de départ contenant au moins 90 % de stéarate et/ou de palmitate de méthyle ou au moins 90 % de suif hydrogéné et méthylé ou d'huile de palme hydrogénée et méthylée.

Il va de soi que des modifications peuvent être apportées aux modes de réalisation qui viennent d'être décrits, notamment par substitution de moyens techniques équivalents, sans sortir pour cela du cadre de la présente invention.

REVENDICATIONS

1 - Procédé de préparation en continu d'acides sulfoniques par sulfonation d'un produit de départ choisi dans le groupe constitué par les esters méthyliques d'acides gras saturés et leur mélange, caractérisé par le fait qu'après avoir soumis ledit produit de départ à l'action d'un agent sulfonant on extrait les acides sulfoniques ainsi obtenus, après un dégazage destiné à éliminer l'agent sulfonant dissous, par traitement répété avec du méthanol légèrement aqueux pour obtenir d'une part, une phase contenant le produit de départ non sulfoné que l'on recycle vers l'étape de sulfonation, et, d'autre part, une phase qui renferme les acides sulfoniques et qui est soumise à une évaporation afin de séparer le méthanol des acides sulfoniques.

2 - Procédé selon la revendication 1, caractérisé en ce que le méthanol aqueux utilisé contient au plus 20 % d'eau et de préférence de 1 à 5 % d'eau.

3 - Procédé selon la revendication 1 ou 2, caractérisé en ce que l'extraction au méthanol aqueux est réalisée à trois étages au moins.

4 - Procédé selon la revendication 3, caractérisé en ce que l'extraction est une extraction à co-courant à trois étages.

5 - Procédé selon la revendication 3, caractérisé en ce que l'extraction est une extraction à contre-courant à cinq étages.

6 - Procédé selon l'une quelconque des revendications 1 à 5, caractérisé en ce que la sulfonation se fait par sulfo-oxydation sous irradiation.

7 - Procédé selon l'une quelconque des revendications 1 à 5, caractérisé en ce que l'agent de sulfonation utilisé est l'anhydride sulfurique.

8 - Procédé selon l'une quelconque des revendications 1 à 7, caractérisé en ce que l'agent sulfonant séparé par dégazage et le méthanol séparé par évaporation sont recyclés respectivement dans l'étape de sulfonation et dans l'étape de mélange avec le produit de sulfonation.

9 - Procédé selon l'une quelconque des revendications 1 à 8, caractérisé en ce que les esters gras utilisés comme produits de départ sont obtenus par hydrogénation et méthylation des huiles et des graisses naturelles d'origine animale ou végétale.

10 - Procédé selon la revendication 9, caractérisé en ce qu'on utilise un produit de départ contenant au moins 90 % d'huile  de palme hydrogénée et méthylée ou de suif hydrogéné et méthylé.

11 - Procédé selon la revendication 10, caractérisé en ce qu'on utilise un produit de départ contenant au moins 90 % de stéarate, palmitate ou laurate de méthyle ou d'un mélange de tels esters.

**Office européen
des brevets**

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée |
|---|---|---|
| | <u>US - A - 2 280 118</u> (BERNARD A. DOMBROW) <br><br> * Page 2, 'claim' 1 - page 3, 'claim' 3, 'claim' 8; page 2, colonne de gauche, lignes 6-11, 32-60 * <br><br> -- | 1-11 |
| | <u>FR - A - 1 547 452</u> (SOC. NAT. DES PETROLES D'AQUITAINE) <br><br> * Page 3, colonne de droite, résumé I; colonne de gauche, lignes 6-18 * <br><br> -- | 1-11 |
| DA | <u>US - A - 3 083 146</u> (WILLIAM M. SWEENEY) <br><br> * Colonne 2, ligne 66 - colonne 3, ligne 7 * <br><br> ---- | 1-11 |

**CLASSEMENT DE LA DEMANDE (Int. Cl. 3)**

C 07 C 143/12
143/18

**DOMAINES TECHNIQUES RECHERCHES (Int. Cl. 3)**

C 07 C 143/00
139/00

**CATEGORIE DES DOCUMENTS CITES**

X: particulièrement pertinent

A: arrière-plan technologique

O: divulgation non-écrite

P: document intercalaire

T: théorie ou principe à la base
   de l'invention

E: demande faisant interférence

D: document cité dans
   la demande

L: document cité pour d'autres
   raisons

&: membre de la même famille,
   document correspondant

Le présent rapport de recherche a été établi pour toutes les revendications

| Lieu de la recherche | Date d'achèvement de la recherche | Examinateur |
|---|---|---|
| La Haye | 06-04-1981 | SUTER |

OEB Form 1503.1   06.78